(19) Europäisches Patentamt
European Patent Office
Office européen des brevets

(11) Veröffentlichungsnummer: **0 045 431**
**A1**

(12) **EUROPÄISCHE PATENTANMELDUNG**

(21) Anmeldenummer: **81105682.9**

(22) Anmeldetag: **20.07.81**

(51) Int. Cl.³: **C 07 C 17/14**
C 07 C 21/24, C 07 C 79/12
C 07 C 79/36, C 07 B 9/00
C 07 C 45/43, C 07 C 47/52
C 07 C 43/225

(30) Priorität: **01.08.80 DE 3029366**

(43) Veröffentlichungstag der Anmeldung:
**10.02.82 Patentblatt 82/6**

(84) Benannte Vertragsstaaten:
**BE CH DE FR GB IT LI**

(71) Anmelder: **BAYER AG**
**Zentralbereich Patente, Marken und Lizenzen**
**D-5090 Leverkusen 1, Bayerwerk(DE)**

(72) Erfinder: **Blank, Heinz Ulrich, Dr.**
**Am Geusfelde 35**
**D-5068 Odenthal(DE)**

(72) Erfinder: **Wolters, Erich, Dr.**
**Streffenweg 22**
**D-5162 Niederzier(DE)**

(72) Erfinder: **Rottloff, Günther**
**Semmelweisstrasse 70**
**D-5000 Koeln 80(DE)**

(72) Erfinder: **Diehl, Herbert, Dr.**
**Heymannstrasse 40**
**D-5090 Leverkusen 1(DE)**

(54) **Verfahren zur Seitenkettenbromierung von Toluolen und Gemische von in der Seitenkette verschieden hoch bromierten Toluolen.**

(57) Substituierte Toluole werden in der Seitenkette bromiert durch Umsetzung mit 0,5 bis 3,2 Mol Brom pro Mol substituiertes Toluol bei 100 bis 250°C, wobei man gegebenenfalls in Gegenwart eines Lösungsmittels arbeitet und wobei man den gleichzeitig gebildeten Bromwasserstoff kontinuierlich aus dem Reaktionsgemisch entfernt. In Abhängigkeit von der eingesetzten Menge Brom werden hierbei Gemische erhalten, die 90 bis 93 Gew.-%, bezogen auf das Gesamtgewicht des Gemisches, an substituiertem Benzalbromid neben geringen Mengen substituiertem Benzylbromid und substituiertem Benzotribromid enthalten, oder Gemische, die 15 bis 95 Gew.-% substituiertes Benzotribromid neben 5 bis 85 Gew.-% substituiertem Benzalbromid, alles bezogen auf das Gemsatgewicht des Gemisches, enthalten oder 40 bis 85 Gew.-% substituiertes Benzalbromid neben 15 bis 60 Gew.-% Benzylbromid, alles bezogen auf das Gesamtgewicht des Gemisches, enthalten. Weiterhin können Gemische erhalten werden, die 95 bis nahe 100 Gew.-% substituiertes Benzalbromid neben nahe 0 bis 5 Gew.-% substituiertem Benzylbromid enthalten. Die erhaltenen substituierten Benzalbromide oder Gemische, die überwiegend solche Benzalbromide enthalten, können zur Herstellung der zugehörigen Benzaldehyde durch Verseifung der Benzalbromidgruppe verwendet werden.

EP 0 045 431 A1

BAYER AKTIENGESELLSCHAFT 5090 Leverkusen, Bayerwerk
Zentralbereich Ha/Zar
Patente, Marken und Lizenzen

# Verfahren zur Seitenkettenbromierung von Toluolen und Gemische von in der Seitenkette verschieden hoch bromierten Toluolen

Die Erfindung betrifft ein Verfahren zur Seitenkettenbromierung von Toluolen und Gemische, die substituiertes Benzylbromid, substituiertes Benzalbromid und substituiertes Benzotribromid enthalten.

Aus der DE-OS 26 12 115 ist es bekannt, m-Phenoxytoluol mit Brom in Gegenwart von Ultraviolettlicht bei 200°C zu m-Phenoxy-benzylbromid umzusetzen.

Aus der DD-PS 118 609 ist die Bromierung von o-Nitrotoluol zum o-Nitrobenzylbromid mit Brom in Tetrachlorkohlenstoff bei Siedehitze bekannt, wobei bei Bestrahlung mit Licht oder bei Katalyse durch Dibenzoylperoxid gearbeitet wird.

Es wurde nun ein Verfahren zur Seitenkettenbromierung von substituierten Toluolen gefunden, das dadurch gekennzeichnet ist, daß man substituierte Toluole der Formel

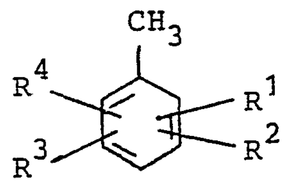

(I) ,

in der

$R^1$ für Wasserstoff oder Halogen steht,

$R^2$ für Wasserstoff, Halogen, die Sulfonsäuregruppe, Sulfonylhalogenid, die Carboxylgruppe, Carbonylhalogenid, Aryloxy oder Aryl steht,

$R^3$ Nitro oder Aryloxy bedeutet und

$R^4$ für Wasserstoff oder Nitro steht,

mit 0,5 bis 3,2 Mol Brom pro Mol gegebenenfalls substituiertes Toluol bei einer Temperatur von 100 bis 250°C, gegebenenfalls in Gegenwart eines Lösungsmittels umsetzt und den gleichzeitig gebildeten Bromwasserstoff kontinuierlich aus dem Reaktionsgemisch entfernt.

Erfindungsgemäß werden substituierte Toluole der Formel (I) der Seitenkettenbromierung unterworfen.

Als Halogen sei beispielsweise Fluor, Chlor oder Brom genannt.

Als Sulfonylhalogenid sei beispielsweise Sulfonylfluorid, Sulfonylchlorid oder Sulfonylbromid, bevorzugt Sulfonylchlorid oder Sulfonylbromid, genannt.

Als Carbonylhalogenid sei beispielsweise Carbonylfluorid, Carbonylchlorid oder Carbonylbromid, bevorzugt Carbonylchlorid oder Carbonylbromid, genannt.

Als Aryloxy sei beispielsweise Phenoxy, 1-Naphthyloxy, 2-Naphthyloxy, 1-Anthryloxy, 2-Anthryloxy oder 9-Anthryloxy sowie Biphenyloxy, bevorzugt Phenoxy, genannt.

Als Aryl sei beispielsweise Phenyl, Naphthyl, Anthryl, Biphenyl, bevorzugt Phenyl, genannt.

Die Arylteile unter den genannten Substituenten können ihrerseits wiederum substituiert sein, beispielsweise durch Halogen, Nitro, Sulfonylhalogenid oder Carbonylhalogenid.

Bevorzugte, erfindungsgemäß einsetzbare Verbindungen der Formel (I) sind substituierte Nitrotoluole der Formel

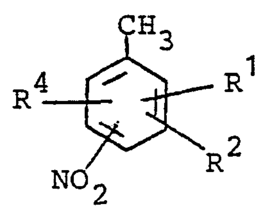

(II) ,

in der

$R^1$, $R^2$ und $R^4$ den genannten Bedeutungsumfang haben.

Unter den Verbindungen der Formel (II) sind solche der Formel

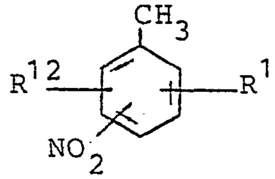

(III) ,

bevorzugt, in der $R^1$ den genannten Bedeutungsumfang hat und
$R^{12}$ für Wasserstoff oder Halogen steht.

Weitere bevorzugte, erfindungsgemäß einsetzbare Verbindungen der Formel (I) sind substituierte Phenoxytoluole der Formel

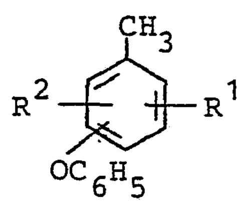
(IV) ,

in der
$R^1$ und $R^2$ den angegebenen Bedeutungsumfang haben.

Besonders bevorzugte substituierte Phenoxytoluole sind solche der Formel

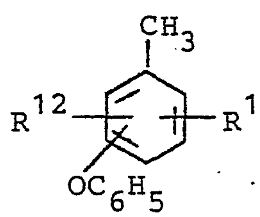
(V) ,

in der
$R^1$ und $R^{12}$ den gegebenen Bedeutungsumfang haben.

Erfindungsgemäß werden zur Seitenkettenbromierung 0,5 bis 3,2 Mol Brom pro Mol substituiertes Toluol eingesetzt. Hierbei kann in Abhängigkeit von der eingesetzten Menge Brom innerhalb des beschriebenen Bereiches ein verschieden hoher Grad an Seitenkettenbromierung erreicht werden. So kann beispielsweise beim Einsatz von etwa 1,8 bis 2,3, vorzugsweise 1,8 bis 2,2, besonders bevorzugt 1,9 - 2,2, Mol Brom pro Mol substituiertes Toluol ein Reaktionsgemisch erhalten werden, das etwa 90 bis 93 Gew.-% substituiertes Benzalbromid der Formel

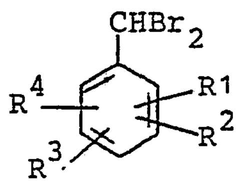

(VI) ,

in der $R^1$ bis $R^4$ die oben genannte Bedeutung haben,

neben untergeordneten Mengen, beispielsweise 3 bis 4 Gew.-%, an Benzylbromid der Formel

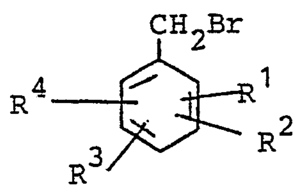

(VII) ,

in der $R^1$ bis $R^4$ die oben genannte Bedeutung haben,

und beispielsweise 4 bis 6 Gew.-% Benzotribromid der Formel

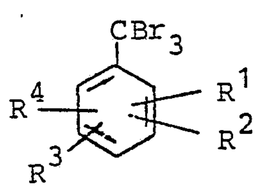

(VIII) ,

in der $R^1$ bis $R^4$ die oben genannte Bedeutung hat,

enthält. Alle Gewichtsprozente beziehen sich auf das Gesamtgewicht des bei der Seitenkettenbromierung erhaltenen Reaktionsgemisches.

Beim Einsatz von etwa 0,5 bis 1,9, bevorzugt 1,2 bis 1,8, besonders bevorzugt 1,5 bis 1,7, Mol Brom pro Mol substituiertes Toluol können beispielsweise Bromierungsgemische erhalten werden, in denen das zugehörige Benzotribromid unter 1 Gew.-%, bevorzugt unter 0,1 Gew.-%, enthalten ist. Beispielsweise kann beim Umsatz mit 1,2 bis 1,8, bevorzugt 1,5 bis 1,7 Mol Brom pro Mol Ausgangsprodukt ein Bromierungsgemisch erhalten werden, das etwa 40 bis 85 Gew.-%, bevorzugt 80 bis 85 Gew.-% des jeweiligen Benzalbromids neben etwa 15 bis 60 Gew.-%, bevorzugt 15 bis 20 Gew.-% des jeweiligen Benzylbromids enthält. Soll beispielsweise das substituierte Benzalbromid als Hauptprodukt erhalten werden, so kann man zur Erhöhung von dessen Ausbeute beispielsweise einen Teil des Bromierungsgemisches, der etwa dem Anteil am zugehörigen Benzylbromid entspricht, beispielsweise durch Destillation trennen und zur weiteren Bromierung wieder in das Ausgangsgemisch zurückführen.

Durch diese Verfahrensvariante gelingt es, die Ausbeute an dem jeweiligen Benzalbromid auf etwa 95 bis nahe 100 Gew.-% des Gesamtgewichts des Bromierungsgemisches zu steigern. Daneben kann dieses Gemisch noch fast 0 bis etwa 5 Gew.-%, bevorzugt weniger als 1 Gew.-%, besonders bevorzugt weniger als 0,1 Gew.-%, an dem jeweiligen Benzylbromid enthalten.

Weiterhin ist es möglich, 1 Mol substituiertes Toluol mit etwa 2,2 bis 3,2 Mol, bevorzugt 2,3 bis 2,7 Mol, besonders bevorzugt 2,4 bis 2,5 Mol, Brom umzusetzen. Die dabei erhaltenen Reaktionsgemische können beispielsweise 10 bis 95 Gew.-%, bevorzugt 20 bis 80 Gew.-%, besonders bevorzugt 30 bis 60 Gew.-%, an substituiertem Benzotribromid und beispielsweise etwa 5 bis 90 Gew.-%, bevorzugt 20 bis 80 Gew.-%, besonders bevorzugt 40 bis 70 Gew.-%, an substituiertem Benzalbromid enthalten.

Nahezu reines Benzotribromid wird beim Einsatz von 2,5 bis 3,2, bevorzugt 2,6 bis 3,1, besonders bevorzugt 2,7 bis 3,0 Mol Brom pro Mol substituiertes Toluol erhalten.

Ein nahezu reines Benzylbromid wird beim Einsatz von 0,5 bis 1,2, bevorzugt 0,6 bis 1,0, besonders bevorzugt 0,7 bis 0,9 Mol Brom pro Mol substituiertes Toluol erhalten.

Das erfindungsgemäße Verfahren wird beispielsweise bei einer Temperatur von 100 bis 250°C durchgeführt. Selbstverständlich können optimale Temperaturbedingungen für die erfindungsgemäße Umsetzung in Abhängigkeit von der Verschiedenartigkeit der genannten Substituenten durch einfache Vorversuche ermittelt werden. Beispielsweise sei für die erfindungsgemäße Umsetzung von Verbindungen der Formel (II) eine Temperatur von 100 bis 200°C, bevorzugt 130 bis 190°C, besonders bevorzugt 140 bis 180°C, genannt. Für die erfindungsgemäße Umsetzung von Verbindungen der Formel (IV) sei beispielsweise eine Temperatur von 130 bis 250°C, bevorzugt 150 bis 220°C, besonders bevorzugt 170 bis 210°C, genannt.

Das erfindungsgemäße Verfahren wird in flüssiger Phase durchgeführt.

Für den Fall, daß in Gegenwart eines Lösungsmittel gearbeitet wird, seien als inerte Lösungsmittel beispielsweise halogenierte aliphatische Kohlenwasserstoffe mit 1 bis 5 Kohlenstoffatomen, wie Methylenchlorid, Chloroform, Tetrachlorkohlenstoff, Dichlorethan, Trichlorethan, Tetrachlorethan und Hexachlorethan, bevorzugt Tetrachlorkohlenstoff, sowie aromatische Kohlenwasserstoffe und deren Halogenderivate, wie Benzol, Chlorbenzol, Brombenzol, Dichlorbenzol, Trichlorbenzol und Tetrachlorbenzol, bevorzugt Chlorbenzol und Dichlorbenzol, sowie Nitrobenzol genannt. Solche Lösungsmittel können sowohl einzeln als auch im Gemisch miteinander für das erfindungsgemäße Verfahren eingesetzt werden. Sie werden beispielsweise in einer Menge von bis zu 10 Vol.-Tl., bevorzugt bis zu 4 Vol.-Tl., besonders bevorzugt bis zu 1 Vol.-Tl., bezogen auf 1 Vol.-Tl. substituiertes Toluol, eingesetzt.

Es ist jedoch auch möglich, das erfindungsgemäße Verfahren ohne Verwendung eines Lösungs- oder Verdünnungsmittels durchzuführen. Wegen der Vermeidung zusätzlicher Trennoperationen ist die Variante ohne zusätzliches Lösungsmittel bevorzugt.

Das Brom kann im erfindungsgemäßen Verfahren in flüssiger, gasförmiger oder in gelöster Form zugegeben werden. Für den Fall, daß das Brom in gelöster Form zugegeben wird, können die oben genannten Lösungsmittel einzeln oder im Gemisch verwendet werden. Für den Fall, daß das Brom gasförmig in das Reaktionsgemisch geleitet wird, kann dies mit oder ohne zusätzliche Inertgaszugabe geschehen. Als hierbei verwendbares Inertgas sei beispielsweise Stickstoff, Bromwasserstoff, Kohlendioxid, Schwefeldioxid, Luft, Wasserstoff, Neon oder Argon genannt.

Der bei der Reaktion des erfindungsgemäßen Verfahrens entstehende Bromwasserstoff wird fortlaufend in kontinuierlicher oder absatzweiser Form aus dem Reaktionssystem abgezogen. Dies geschieht im allgemeinen, wenn bevorzugterweise bei Normaldruck gearbeitet wird, über den aufgesetzten Rückflußkühler und eine nachgeschaltete Absorptionseinrichtung. Für den Fall, daß unter erhöhtem Druck gearbeitet wird, etwa um ein tiefer siedendes Lösungsmittel aus der Gruppe der oben erwähnten Lösungsmittel einzusetzen, wird der Bromwasserstoff über eine Druckschleuse am Kopf des Rückflußkühlers aus dem Reaktor entnommen. Gegen Ende der Reaktion kann es vorteilhaft sein, zur vollständigen Entfernung des Bromwasserstoffs einen Unterdruck an den Reaktor anzulegen, beispielsweise im Bereich von 0,5 bis 0,95 bar. Zur Entfernung des Bromwasserstoffs aus dem Reaktionssystem kann auch zusätzlich ein Inertgas durch das Reaktionsgemisch geleitet werden. Hierfür können die gleichen Inertgase eingesetzt werden, die für die Einleitung von gasförmigem Brom bereits genannt worden sind.

Für den Fall, daß bei Normaldruck oder bei Überdruck gearbeitet wird, kann es auch zweckmäßig sein, das Anlegen von Unterdruck und/oder das Durchleiten eines Inertgases erst nach Beendigung der Reaktion des erfindungsgemäßen Verfahrens vorzunehmen.

In einer weiteren Verfahrensvariante wird der Bromwasserstoff aus dem Reaktionsgemisch dadurch entfernt, daß neben der organischen, gegebenenfalls ein zusätzliches Lösungsmittel enthaltenden Phase eine wäßrige

Phase vorhanden ist, deren pH-Wert bei 1 bis 8, bevorzugt 2 bis 7, besonders bevorzugt 3 bis 6, gehalten wird. Selbstverständlich ist diese Verfahrensvariante nur bei erhöhtem Druck möglich. Hierzu sei beispielsweise ein Druck von 1,5 bis 40 bar genannt. Bevorzugt wird unter dem Eigendruck gearbeitet, der sich bei den gewählten Reaktionstemperaturen einstellt. Zur pH-Einstellung in der Wasserphase können beispielsweise unter Druck zugeführte wäßrige Lösungen von Natriumhydroxid, Kaliumhydroxid, Natriumcarbonat, Kaliumcarbonat eingesetzt werden.

Im allgemeinen wird das erfindungsgemäße Verfahren so durchgeführt, daß man das substituierte Toluol, gegebenenfalls auch in einer technischen Qualität mit einem Gehalt von etwa 95 bis 99 Gew.-% an dem gewünschten substituierten Toluol, gegebenenfalls mit einem inerten Lösungsmittel auf die gewünschte Temperatur aufheizt und bei guter Durchmischung die vorgesehene Menge an Brom in der Weise eindosiert, daß es unterhalb der Oberfläche des Reaktionsgemisches, zweckmäßigerweise in der Nähe des Bodens des Reaktionsgefäßes, eintritt. Dies hat den Vorteil, daß praktisch kein Brom aus dem Reaktionsgemisch entweicht. Es ist auch möglich, nur einen Teil des substituierten Toluols vorzulegen und das restliche substituierte Toluol simultan mit dem Brom in den Reaktor einzudosieren, wobei die simultane Zugabe sowohl chargenweise als auch kontinuierlich durchgeführt werden kann. Nach Zugabe der vorgesehenen Menge an Brom wird bis zum Ende der Bromwasserstoffentwicklung nachgerührt.

Für den Fall, daß das bei der Seitenkettenbromierung erhaltene Gemisch weiter verarbeitet werden soll, ist eine vorherige Aufarbeitung des Reaktionsgemisches bei lösungsmittelfreier Arbeitsweise vor der Weiterverarbeitung im allgemeinen nicht erforderlich. Beispielsweise kann ein Reaktionsgemisch, das 90 bis 93 Gew.-% substituiertes Benzalbromid enthält, in dieser Form gegebenenfalls nach Entfernung von restlichem Bromwasserstoff im Vakuum, direkt weiter verarbeitet werden. Bei Bedarf kann selbstverständlich eine weitere Reinigung oder auch eine Fraktionierung des Reaktionsgemisches durch Destillation, Vakuumdestillation oder Umkristallisation erfolgen. Das erfindungsgemäße Verfahren kann sowohl kontinuierlich als auch diskontinuierlich durchgeführt werden.

Die Erfindung betrifft weiterhin Gemische, die 90 bis 93 Gew.-% substituiertes Benzalbromid der Formel (VI), 3 bis 4 Gew.-% substituiertes Benzylbromid der Formel (VII) und 4 bis 6 Gew.-% substituiertes Benzotribromid der Formel (VIII) enthalten, wobei die Gewichtsprozente sich auf das Gesamtgewicht der Gemische beziehen.

Die Erfindung betrifft weiterhin Gemische, die 15 bis 95 Gew.-% substituiertes Benzotribromid der Formel (VIII) und 5 bis 85 Gew.-% substituiertes Benzalbromid der Formel (VI) enthalten, wobei sich die Gewichtsprozente auf das Gesamtgewicht der Gemische beziehen.

Die Erfindung betrifft weiterhin Gemische, die 40 bis 85 Gew.-% substituiertes Benzalbromid der Formel (VI) und 15 bis 60 Gew.-% substituiertes Benzylbromid der Formel (VII) enthalten, wobei die Gewichtsprozente sich auf das Gesamtgewicht der Gemische beziehen.

Die Erfindung betrifft weiterhin Gemische, die 95 bis nahe 100 Gew.-% substituiertes Benzalbromid der Formel (VI) und nahezu 0 bis etwa 5 Gew.-% substituiertes Benzylbromid der Formel (VII) enthalten, wobei die Gewichtsprozente auf das Gesamtgewicht der Gemische bezogen sind.

Das erfindungsgemäße Verfahren wird bevorzugt zur Herstellung der substituierten Benzalbromide der Formel (VI) durch Umsetzung von 1,8 bis 2,3 Mol Brom pro Mol substituiertes Toluol der Formel (I) durchgeführt.

Besonders bevorzugt sei die Herstellung von Nitrobenzalbromiden der Formel

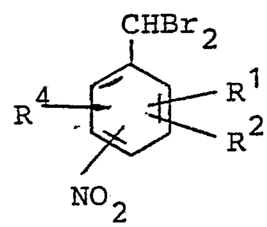

(IX) ,

in der $R^1$, $R^2$ und $R^4$ die gegebene Bedeutung haben, genannt. Ganz besonders bevorzugt ist hierbei die Herstellung von 4-Nitrobenzalbromid.

Weiterhin besonders bevorzugt sei die Herstellung von Phenoxy-benzalbromiden der Formel

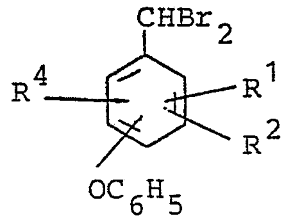

(X) ,

in der $R^1$, $R^2$ und $R^4$ die genannte Bedeutung haben, genannt. Ganz besonders bevorzugt unter diesen sind die gegebenenfalls substituierten 3-Phenoxy-benzalbromide, die gegebenenfalls im Rahmen der obigen Beschreibung auch im zweiten Arylkern substituiert sein können.

Die erfindungsgemäß erhältlichen Bromierungsprodukte der substituierten Toluole und die genannten erfindungsgemäßen Gemische sind wertvolle Zwischenprodukte, die durch Verseifung des in der Methylgruppe erfindungsgemäß eingetretenen Broms den Zugang zu wertvollen Verbindungen eröffnen. So kann beispielsweise ein erfindungsgemäß erhältliches substituiertes Benzalbromid oder die erfindungsgemäßen Gemische, die überwiegend Benzalbromid enthalten, durch Verseifung in die zugehörigen substituierten Benzaldehyde umgewandelt werden. Diese Benzaldehyde sind beispielsweise wertvolle Ausgangsprodukte für Riechstoffe. Weiterhin können die erfindungsgemäß erhältlichen substituierten Benzylbromide in die zugehörigen Benzylalkohole umgewandelt werden, die durch Veresterung ebenfalls zu Riech- oder Aromastoffen, zu Pharmazeutika und anderen Wirkstoffen, wie Insektiziden, Fungiziden und Bakteriziden umgewandelt werden können. Die erfindungsgemäß erhältlichen substituierten Benzotribromide bzw. die erfindungsgemäßen Gemische mit einem überwiegenden Gehalt an Benzotribromid können beispielsweise durch Verseifung in die zugehörigen substituierten Benzoesäuren oder Benzoesäurebromide umgewandelt werden, die ebenfalls wertvolle Ausgangsstoffe für die Herstellung von Riechstoffen, biologischen

Wirkstoffen, wie Pharmazeutika, Insektiziden, Bakteriziden und Fungiziden, sind oder für den Fall der Benzoesäuren als Komponente für Lackrohstoffe und Alkydharze verwendbar sind.

Beispielsweise werden substituierte Benzaldehyde als Zwischenprodukt zur Herstellung von koronar-dilatatorisch und peripher-dilatatorisch wirksamen, blutdrucksenkenden Pharmazeutika (nach DE-OS 19 63 118) eingesetzt.

Des weiteren sei beispielsweise der Einsatz von 3-Phenoxybenzylalkoholen, die durch Verseifung der erfindungsgemäß dargestellten zugehörigen Benzylbromide erhalten werden, und der Einsatz von 3-Phenoxy-$\alpha$-cyano-benzylalkoholen, die durch dem Fachmann bekannte Anlagerung von Cyanwasserstoff an die erfindungsgemäß erhältlichen zugehörigen Benzaldehyde erhalten werden, bei der Herstellung von pyrethroiden Insektiziden genannt (DE-OS 25 47 534). Nach DE-OS 27 30 515 können an Stelle der genannten Benzylalkohole auch die zugehörigen Benzylbromide für die Herstellung von Pyrethroiden eingesetzt werden. Nitrobenzaldehyde, die aus den zugehörigen Benzalbromiden durch Verseifung zugänglich sind, sind beispielsweise Zwischenprodukte für Farbstoffe der verschiedensten Klassen (Ullmann's Enzyklopädie der Technischen Chemie, 3. Auflage, 4. Band, S. 241, Verlag Urban und Schwarzenberg, München-Berlin 1953).

Die Erfindung betrifft daher weiterhin die Verwendung der erfindungsgemäß erhältlichen substituierten Benzylbromide, Benzalbromide und Benzotrichloride sowie die Verwendung der erfindungsgemäßen Gemische für die Herstellung der zugehörigen Benzylalkohole, Benzaldehyde und Benzoesäuren bzw. Benzylbromiden der Formeln



BAD ORIGINAL

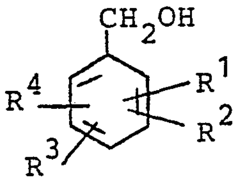 (XI) , 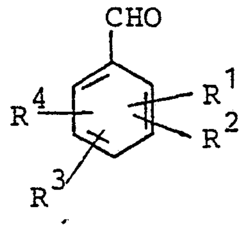 (XII) ,

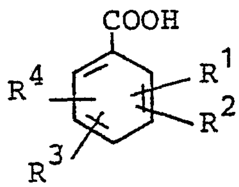 (XIII) und 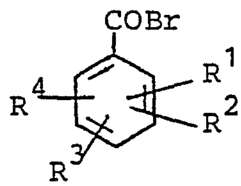 (XIV) ,

in denen $R^1$ bis $R^4$ die oben genannte Bedeutung haben.

Die Erfindung betrifft besonders die Verwendung der Benzalbromide bzw. der erfindungsgemäßen Gemische, die überwiegend solche Benzalbromide enthalten, zur Herstellung der zugehörigen Benzaldehyde. Die Erfindung betrifft insbesondere die Verwendung von gegebenenfalls substituierten 3-Phenoxybenzalbromiden zur Herstellung der zugehörigen Benzaldehyde. Die Verseifung der erfindungsgemäß erhältlichen Benzalbromide bzw. der erfindungsgemäßen Gemische, die überwiegend Benzalbromide enthalten, kann beispielsweise mit Säuren, wie 50 bis 90 gew.-%iger Schwefelsäure oder 50 bis 90 gew.-%iger Ameisensäure bei erhöhter Temperatur durchgeführt werden.

Die Erfindung betrifft ferner die Verwendung der oben geschilderten Benzalbromide oder der Gemische, die überwiegend Benzalbromide enthalten, zur Herstellung der zugehörigen Benzaldehyde, die dadurch gekennzeichnet ist, daß man eine Aufschlämmung des Benzaldehydes und eines Salzes einer schwachen Säure in Wasser unter erhöhtem Druck auf eine Temperatur von 100 bis 250°C erhitzt. Als Benzalbromide seien beispielsweise die der



BAD ORIGINAL

Formel (VI) genannt. Weiterhin können solche bereits oben beschriebene Gemische eingesetzt werden, die überwiegend Benzalbromid enthalten. Darüberhinaus können die hier einsetzbaren Benzaldehyde auch teilweise oder ganz Chlor anstelle von Brom enthalten.

Als Salze einer schwachen Säure seien beispielsweise Salze der Kohlensäure, der Borsäure, der Essigsäure, bevorzugt Salze der Kohlensäure, genannt. Bevorzugt werden wasserunlösliche Salze der Kohlensäure eingesetzt, beispielsweise die Erdalkalicarbonate, wie Magnesiumcarbonat, Calciumcarbonat, Strontiumcarbonat, Bariumcarbonat, bevorzugt Magnesiumcarbonat oder Calciumcarbonat. Weiterhin einsetzbar ist beispielsweise das Magnesiumoxid, das als unlösliches neutrales Salz der schwachen Säure Wasser angesehen werden kann.

Bei dieser Umsetzung des Benzalbromides zum Benzaldehyd wird ein pH-Wert von beispielsweise 3 bis 8, bevorzugt 4 bis 7, besonders bevorzugt 5 bis 7, eingehalten.

Die Temperatur beträgt hierbei beispielsweise 100 bis 250°C, bevorzugt 130 bis 200°C, besonders bevorzugt 140 bis 180°C.

Selbstverständlich ist es auch möglich, lösliche Salze beispielsweise die Alkalisalze der Kohlensäure einzusetzen, wie Natriumcarbonat, Kaliumcarbonat, Natriumhydrogencarbonat oder Kaliumhydrogencarbonat.

Die Verseifung des Benzalbromids zum Benzaldehyd wird in Gegenwart von Wasser in heterogener Phase durchgeführt. Die Menge Wasser ist hierbei in weiten Grenzen



BAD ORIGINAL

variabel, beispielsweise seien 50 bis 1000 Gew.-%, bevorzugt 100 bis 600, besonders bevorzugt 150 bis 400 Gew.-% Wasser, bezogen auf die Gesamtmenge an umzusetzendem Benzaldehyd und dem Salz der schwachen Säure, genannt.

Die Menge des Salzes der schwachen Säure beträgt hierbei beispielsweise 0,8 bis 5, bevorzugt 0,9 bis 3, besonders bevorzugt 1 bis 2 Kationenäquivalente des Salzes der schwachen Säure pro Bromidäquivalent aus dem Benzalbromid.

Das Verfahren zur Verseifung des Benzalbromides zum Benzaldehyd wird in einem Druckreaktor durchgeführt. Die Einstellung des pH-Wertes innerhalb des beschriebenen Bereiches geschieht durch Dosierung der Menge des Salzes der schwachen Säure entsprechend dem Verlauf der Hydrolyse. Hierbei kann beispielsweise die gesamte Menge des Benzalbromides vorgelegt werden und die Menge des Salzes der schwachen Säure im Verlaufe der Hydrolyse zugegeben werden. Es kann jedoch auch nur ein Teil des Benzalbromides und des Salzes der schwachen Säure vorgelegt und reagiert werden und im Verlaufe der Reaktion weiteres Benzalbromid und weiteres Salz der schwachen Säure kontinuierlich oder chargenweise, gegebenenfalls simultan zugegeben werden. Nach Beendigung der Reaktion wird der Druckreaktor abgekühlt, entspannt und entleert. Der gewünschte Benzaldehyd kann im allgemeinen durch Filtration oder Extraktion aus dem Reaktionsgemisch isoliert werden und nach üblichen Mitteln, beispielsweise durch Umkristallisation oder durch Vakuumdestillation, weiter gereinigt werden.



BAD ORIGINAL

Beispiel 1

In einem Reaktionskolben, der mit Tropftrichter, dessen Eintropfrohr bis an den Boden des Kolbens reicht, Rührer und Rückflußkühler mit Gasableitungsrohr ausgerüstet ist, werden 109,6 g 4-Nitrotoluol (0,8 Mol) unter Rühren auf 150°C aufgeheizt. Im Verlauf von etwa 7 Stunden werden 235 g Brom (1,47 Mol) so zugetropft, daß im Rückflußkühler kein Brom erscheint. Es wird noch bis zum Ende der Gasentwicklung nachgerührt (etwa weitere 15 Minuten). Es werden etwa 230 g eines Reaktionsgemisches erhalten, das beim Abkühlen bei etwa 60°C erstarrt und nach gaschromatographischer Analyse folgende Stoffe enthält: 10,9 Gew.-% 4-Nitrobenzylbromid, 85,9 Gew.-% 4-Nitrobenzalbromid und 2,5 Gew.-% 4-Nitrobenzotribromid. Das entspricht einer Ausbeute von 84 % der theoretischen Ausbeute an 4-Nitrobenzalbromid. 40 g dieses rohen Reaktionsgemisches werden in 300 ml Methanol aufgelöst und zum Sieden erhitzt. Bei der Siedehitze werden 150 ml Wasser hinzugefügt. Anschließend wird von etwas ungelöstem Material abgesaugt. Man läßt auf 5°C abkühlen und saugt den entstandenen Kristallbrei ab. Nach dem Trocknen erhält man 33 g eines etwa 97 %igen 4-Nitrobenzalbromids mit einem Schmelzpunkt von 76 bis 78°C.

Beispiel 2

In einer Apparatur wie in Beispiel 1 werden 141,4 g 3-Phenoxy-4-fluortoluol und 235,2 g Brom (1,47 Mol) bei einer Temperatur von 200°C in der in Beispiel 1

beschriebenen Weise umgesetzt. Man erhält 256,6 g eines Bromierungsgemisches, das nach gaschromatographischer Analyse 89,3 Gew.-% 3-Phenoxy-4-fluorbenzalbromid, 2,1 Gew.-% 3-Phenoxy-4-fluor-benzylbromid und 4,4 Gew.-% 3-Phenoxy-4-fluor-benzotribromid enthält. Das Rohprodukt hat einen Siedepunkt von 140 bis 145°C (0,2 bis 0,3 mbar).

Beispiel 3

In einem 500 ml-4-Halskolben mit Rührer, Rückflußkühler, Thermometer, Kapillare und Anschluß an die Wasserstrahlpumpe werden 40 g etwa 97 %iges 4-Nitrobenzalbromid (0,132 Mol) und 320 g 96 %ige Schwefelsäure vorgelegt. Unter Rühren wird bei voll angelegtem Wasserstrahlpumpen - Vakuum durch die Kapillare Stickstoff eingeleitet. Dann wird das Gemisch auf 90°C aufgeheizt, wobei das Benzalbromid in Lösung geht. Das Vakuum im Reaktionskolben beträgt 150 bis 200 mbar. Nach 10 Stunden ist das gesamte Benzalbromid umgesetzt. Die rotbraune Lösung wird nach dem Abkühlen auf Eis gegossen, 30 Minuten gerührt und abgesaugt. Der Filterkuchen wird neutral gewaschen und bei 50°C im Vakuum getrocknet. Man erhält 19 g eines beigefarbenen Produktes vom Schmelzpunkt 101 bis 103°C, das folgende Verbindungen enthält:
92,9 Gew.-% 4-Nitrobenzaldehyd und 3,6 Gew.-% 4-Nitrobenzoesäure. Dies entspricht einer Ausbeute von etwa 95 % der theoretischen Ausbeute.

Beispiel 4

Ein 0,7 l-V4A-Autoklav mit Rührer (400 Upm) wird mit 40 g etwa 97 %igem 4-Nitrobenzalbromid (0,132 Mol), 170 g Wasser und 30 g Calciumcarbonat (0,3 Mol) beschickt, mit Stickstoff gespült und unter Rühren auf 150°C erhitzt. Hierbei entsteht, unter anderem durch freigesetztes $CO_2$, ein Druck von 13,5 bar. Nach 5 Stunden wird der Autoklav abgekühlt, entspannt und entleert. Das Reaktionsgemisch wird abgesaugt und der Filterkuchen zweimal mit jeweils 100 ml heißem Methanol extrahiert. Das Lösungsmittel wird verdampft und der Rückstand bei 50°C im Vakuum getrocknet. Man erhält 21 g eines gelblichen Produktes vom Schmelzpunkt 103 bis 104°C, das 93,4 Gew.-% 4-Nitrobenzaldehyd und 0,4 bis 0,5 Gew.-% 4-Nitrobenzoesäure enthält. Dies entspricht einer Ausbeute von etwa 98 % der theoretischen Ausbeute.

Beispiel 5

72 g 94,9 gew.-%iges 3-Phenoxy-4-fluor-benzalbromid (0,19 Mol) werden mit 32,5 g 85 gew.-%iger Ameisensäure (0,6 Mol) bei Rückflußbedingungen (etwa 105 bis 120°C) während 14 Stunden erhitzt. Anschließend wird die organische Phase abgetrennt, mit 100 ml Wasser durchgerührt und mit konzentrierter wäßriger Sodalösung neutral gestellt. Die organische Phase wird abgetrennt und über Natriumsulfat getrocknet. Zur Erhöhung der Ausbeute wird die wäßrige Phase mit wenig Toluol ausgeschüttelt und das Trockenmittel Natriumsulfat ebenfalls mit Toluol gewaschen. Die erhaltenen Lösungen in Toluol werden vereinigt und vom Toluol befreit. Der

Gesamtrückstand, der erhalten wird, beträgt 44,3 g und enthält nach gaschromatographischer Analyse 92,7 Gew.-% 3-Phenoxy-4-fluor-benzaldehyd. Dies entspricht 100 % der theoretischen Ausbeute. Durch Destillation bei 110 bis 112°C (0,3 bis 0,4 mbar) werden 34 g eines Destillats erhalten, das nach gaschromatographischer Analyse 96,3 Gew.-% des 3-Phenoxy-4-fluor-benzaldehyds enthält.

Patentansprüche:

1) Verfahren zur Seitenkettenbromierung von substituierten Toluolen, dadurch gekennzeichnet, daß man substituierte Toluole der Formel

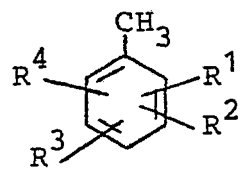

,

in der

$R^1$ für Wasserstoff oder Halogen steht,

$R^2$ Wasserstoff, Halogen, die Sulfonsäuregruppe, Sulfonylhalogenid, die Carboxylgruppe, Carbonylhalogenid, Aryloxy oder Aryl bedeutet,

$R^3$ Nitro oder Aryloxy bedeutet und

$R^4$ für Wasserstoff oder Nitro steht,

mit 0,5 bis 3,2 Mol Brom pro Mol substituiertes Toluol bei einer Temperatur von 100 bis 250°C, gegebenenfalls in Gegenwart eines Lösungsmittels umsetzt und den gleichzeitig gebildeten Bromwasserstoff kontinuierlich aus dem Reaktionsgemisch entfernt.

2) Verfahren nach Anspruch 1, dadurch gekennzeichnet, daß man substituierte Nitrotoluole der Formel

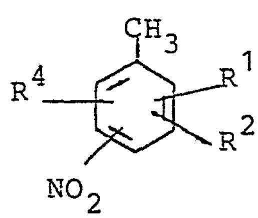

einsetzt,

in der

$R^1$, $R^2$ und $R^4$ die in Anspruch 1 gegebene Bedeutung haben.

3) Verfahren nach Anspruch 1, dadurch gekennzeichnet, daß man substituierte Phenoxy-toluole der Formel

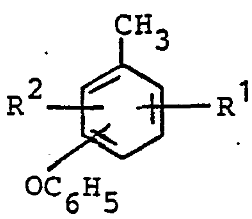

einsetzt,

in der

$R^1$ und $R^2$ die in Anspruch 1 angegebene Bedeutung haben.

4) Verfahren nach Anspruch 1 bis 3, dadurch gekennzeichnet, daß mit 1,8 bis 2,3 Mol Brom gearbeitet wird.

5) Verfahren nach Anspruch 1 bis 3, dadurch gekennzeichnet, daß mit 0,5 bis 1,9 Mol Brom gearbeitet wird.

6) Verfahren nach Anspruch 1 bis 3, dadurch gekennzeichnet, daß mit 2,2 bis 3,2 Mol Brom gearbeitet wird.

7) Gemische, enthaltend 90 bis 93 Gew.-% substituiertes Benzalbromid der Formel

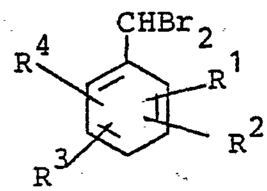

,

3 bis 4 Gew.-% substituiertes Benzylbromid der Formel

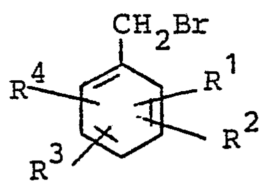

,

und 4 bis 6 Gew.-% substituiertes Benzotribromid der Formel

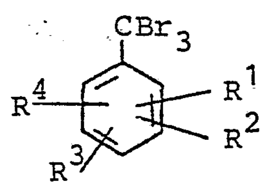

,

alles bezogen auf das Gesamtgewicht der Gemische, worin

$R^1$ für Wasserstoff oder Halogen steht,

$R^2$ für Wasserstoff, Halogen, die Sulfonsäuregruppe, Sulfonylhalogenid, die Carboxylgruppe, Carbonylhalogenid, Aryloxy oder Aryl steht,

$R^3$ Nitro oder Aryloxy bedeutet und

$R^4$ für Wasserstoff oder Nitro steht.

8) Gemische, enthaltend 15 bis 95 Gew.-% substituiertes Benzotribromid der Formel

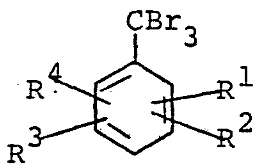

und 5 bis 85 Gew.-% substituiertes Benzalbromid der Formel

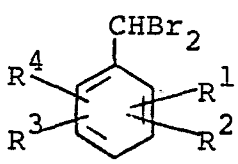

,

bezogen auf das Gesamtgewicht der Gemische, wobei $R^1$ bis $R^4$ die in Anspruch 7 gegebene Bedeutung haben.

9) Gemische, enthaltend 40 bis 85 Gew.-% substituiertes Benzalbromid der Formel

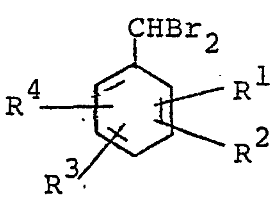

und 15 bis 60 Gew.-% Benzylbromid der Formel

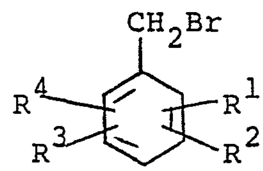

bezogen auf das Gesamtgewicht der Gemische, wobei $R^1$ bis $R^4$ die in Anspruch 7 gegebene Bedeutung haben.

10) Gemische, enthaltend 95 bis nahe 100 Gew.-% substituiertes Benzalbromid der Formel

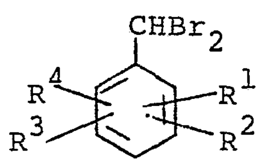

und nahe 0 bis 5 Gew.-% substituiertes Benzylbromid der Formel

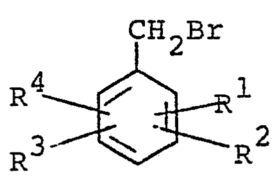

bezogen auf das Gesamtgewicht der Gemische, wobei $R^1$ bis $R^4$ die in Anspruch 7 gegebene Bedeutung haben.

11) Verwendung der substituierten Benzalbromide der Formel

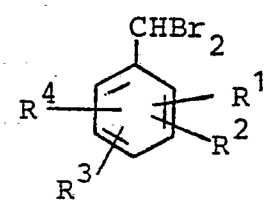

,

in der
$R^1$ für Wasserstoff oder Halogen steht,
$R^2$ Wasserstoff, Halogen, die Sulfonsäuregruppe, Sulfonylhalogenid, die Carboxylgruppe, Carbonylhalogenid, Aryloxy oder Aryl bedeutet,
$R^3$ Nitro oder Aryloxy bedeutet und
$R^4$ für Wasserstoff oder Nitro steht

und der Gemische, die überwiegend solche Benzalbromide enthalten zur Herstellung der zugehörigen Benzaldehyde durch Verseifung der Benzalbromidgruppe.

12) Verwendung der Benzalbromide nach Anspruch 11 zur Herstellung der zugehörigen Benzaldehyde durch Verseifung, dadurch gekennzeichnet, daß die Verseifung bei 100 bis 250°C unter erhöhtem Druck in Gegenwart von Wasser mit Hilfe von Salzen schwacher Säuren bei einem pH-Wert von 3 bis 8 durchgeführt wird.

13) Verfahren nach Anspruch 1, dadurch gekennzeichnet, daß zur Herstellung der substituierten Benzalbromide der Formel

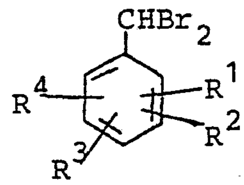

,

in der $R^1$ bis $R^4$ die in Anspruch 1 gegebene Bedeutung haben,
1,8 bis 2,3 Mol Brom pro Mol substituiertes Toluol eingesetzt werden.

14) Verfahren nach Anspruch 1, 2 und 13, dadurch gekennzeichnet, daß man zur Herstellung der substituierten Nitrobenzaldehyde substituierte Nitro-toluole einsetzt.

15) Verfahren nach Anspruch 1, 2 und 13, dadurch gekennzeichnet, daß man zur Herstellung der substituierten 3-Phenoxy-benzalbromide substituierte 3-Phenoxy-toluole einsetzt.

0045431

Europäisches Patentamt

# EUROPÄISCHER RECHERCHENBERICHT

Nummer der Anmeldung

EP 81 10 5682.9

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | EP - A1 - 0 011 288 (HOECHST)<br>* Anspruch 3 *<br>-- | 1,6 |
| | DE - B2 - 2 841 664 (ETHYL CORP.)<br>* Anspruch *<br>-- | 1,3, 15 |
| | DE - A1 - 2 857 580 (HOECHST)<br>* Beispiel 2 *<br>-- | 1 |
| A | DE - A1 - 2 752 612 (BAYER)<br>* Ansprüche 1, 3, 4; Beispiele *<br>-- | 12 |
| P | DE - A1 - 2 926 021 (BAYER)<br>* Anspruch 1; Beispiele *<br>---- | 12 |

KLASSIFIKATION DER ANMELDUNG (Int. Cl.³)

C 07 C 17/14
C 07 C 21/24
C 07 C 79/12
C 07 C 79/36
C 07 B 9/00
C 07 C 45/43
C 07 C 47/52
C 07 C 43/225

RECHERCHIERTE SACHGEBIETE (Int. Cl.³)

C 07 B 9/00
C 07 C 17/14
C 07 C 21/24
C 07 C 43/225
C 07 C 45/43
C 07 C 47/52
C 07 C 47/54
C 07 C 47/542
C 07 C 47/55
C 07 C 79/12
C 07 C 79/36

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| Berlin | 03-11-1981 | KNAACK |

EPA form 1503.1 06.78